# EUROPEAN PATENT SPECIFICATION

(11) **EP 3 050 872 B1**
(45) Date of publication and mention of the grant of the patent: **03.10.2018**
(21) Application number: 15305097.6
(22) Date of filing: 28.01.2015
(51) Int. Cl.: C07C 311/09, H01G 11/60, H01M 10/052, H01M 10/0569

(54) **Fluorinated sulfonamide as electrolyte (co-)solvent for lithium-ion batteries**
Fluoriertes Sulphonamid als Elektrolyt-(Co-)Lösungsmittel für Lithium-Ionenbatterien
Sulfonamide fluoré comme (co-) solvant électrolyte pour batteries lithium-ion

(43) Date of publication of application: 03.08.2016
(73) Proprietor: Westfälische Wilhelms-Universität Münster, 48149 Münster (DE); Institut Polytechnique de Grenoble, 38031 Grenoble Cedex (FR); Centre National de la Recherche Scientifique (C.N.R.S.), 75794 Paris Cedex 16 (FR)
(72) Inventor: Bresser, Dominic, 89073 Ulm (DE); Passerini, Stefano, 89073 Ulm (DE); Kalhoff, Julian, 48149 Münster (DE); Alloin, Fannie, 38220 Vizille (FR); Bolloli, Marco, 38100 Grenoble (FR); Sanchez, Jean-Yves, 38330 Saint-Ismier (FR)
(74) Representative: Michalski Hüttermann & Partner Patentanwälte mbB

(56) References cited:
- EP-A1- 1 088 814
- ILYA&EMSP14;M. LYAPKALO ET AL: "Study of Unusually High Rotational Barriers about SN Bonds in Nonafluorobutane-1-sulfonamides: The Electronic Nature of the Torsional Effect", HELVETICA CHIMICA ACTA, vol. 85, no. 12, 1 December 2002 (2002-12-01), pages 4206-4215, XP55200417, ISSN: 0018-019X, DOI: 10.1002/hlca.200290006
- FU SHI-TAO ET AL: "N,N-dialkyl perfluoroalkanesulfonamides: Synthesis, characterization and properties", JOURNAL OF FLUORINE CHEMISTRY, ELSEVIER, NL, vol. 147, 17 January 2013 (2013-01-17), pages 56-64, XP028987080, ISSN: 0022-1139, DOI: 10.1016/J.JFLUCHEM.2013.01.009
- E. KRAMER ET AL: "Dependency of Aluminum Collector Corrosion in Lithium Ion Batteries on the Electrolyte Solvent", ECS ELECTROCHEMISTRY LETTERS, vol. 1, no. 5, 29 August 2012 (2012-08-29) , pages C9-C11, XP55200208, ISSN: 2162-8726, DOI: 10.1149/2.004205eel

## Description

The present invention relates to an electrolyte solution for use in lithium-ion batteries and particularly to an electrolyte solvent for use with sulfonimides and other electrolyte salts suffering aluminum current collector corrosion.

Lithium-ion batteries are nowadays the leading battery technology, since they offer efficient and high energy storage as well as high power density, and thus dominate the market for batteries used in portable electronic devices. The state-of-the-art in lithium-ion batteries is the utilization of carbonate-based electrolyte solvents in combination with lithium hexafluorophosphate (LiPF₆) as conducting salt. LiPF6 is the lithium salt of choice due to some well-balanced properties, i.e. good ionic conductivity, a beneficial effect on the solid electrolyte interphase (SEI) formation, as well as the prevention of the aluminium current collector corrosion at elevated potentials. However, LiPF6 suffers from low thermal, electrochemical and chemical stabilities forming highly toxic hydrofluoric acid (HF), degrading the batteries active materials and leading to a capacity and hence a battery life fading. Furthermore, leakage of the electrolyte results in huge safety issues.

Lithium bis(trifluoromethanesulfonyl)imide (LiTFSI) provides a promising alternative as conducting salt since it provides a high ionic conductivity and a significantly improved thermal, chemical and electrochemical stability relatively to LiPF₆. However, LiTFSI induces the oxidative aluminium dissolution, i.e. the so-called "corrosion" of the aluminum current collector in combination with state-of-the-art carbonate electrolyte solvents. It has been proposed that current collector corrosion does not only depend on the electrolyte salt but also on the used electrolyte solvent, such as, for example, in Krämer et al., ECS Electrochemistry Letters, 1(5) C9-C11 (2012).

EP 1 088 814 A1 discloses fluorinated sulfonamides as highly flame-resistant solvents for use in electrochemical cells.

The object underlying the present invention was to provide compounds usable for the suppression or prevention of the aluminum current collector corrosion resulting from the use of LiTFSI and other electrolyte salts suffering aluminum current collector corrosion in electrolytes.

The problem is solved by an electrolyte solution comprising an electrolyte salt and an electrolyte solvent, wherein the electrolyte solvent comprises at least one fluorinated sulfonamide according to the general formula (1) R¹-SO₂-NR²R³ wherein:
- R¹: is selected from the group comprising fluorinated linear C₁-C₄-alkyl, branched C₃-C₄-alkyl, linear C₂-C₄-ether and/or branched C₃-C₄-ether;
- R², R³: are selected, the same or independently of the other, from the group comprising linear C₂-C₅-ether and/or branched C₃-C₁₀-ether.

It was surprisingly found that the fluorinated sulfonamides R¹-SO₂-NR²R³ according to the general formula (1) markedly supress or prevent the aluminum current collector from anodic dissolution. Advantageously, even after 100 potentiodynamic cyclic sweeps using LiTFSI as electrolyte salt and 1,1,2,2,3,3,4,4,4-nonafluoro-N,N-bis(2-methoxyethyl)butane-1-sulfonamide as electrolyte solvent no aluminum corrosion was observed on the aluminum foil current collector. The invention has the advantage of enabeling the utilization of sulfonimide-based lithium salts as electrolyte salt since these conductive salts are chemically, electrochemically, and thermally more stable compared to the state-of-the-art conducting salt LiPF₆ the safety of the overall battery is improved. An enhanced overall safety of the battery is particularly important for the application in large-scale devices as, for instance, electric vehicles. Moreover, sulfonimide-based lithium salts are more stable against high potentials, and thus also enable increased energy densities of such batteries using high voltage cathode materials.

The term "alkyl" according to the invention is to be understood as including straight-chain or linear and branched alkyl groups, if not defined otherwise. The term "C₁-C₄-alkyl" as used herein refers to straight-chain or branched alkyl groups having 1 to 4 carbon atoms.

The term "branched C₃-C₄-alkyl" as used herein refers to branched alkyl groups having 3 to 4 carbon atoms. A branched C₃-C₄-alkyl group may be selected from the group comprising isopropyl, isobutyl (2-methylpropyl), sec-butyl (1-methylpropyl) and/or tert-butyl (1,1-dimethylethyl). Linear or straight-chain C₁-C₄-alkyl groups may be selected from the group comprising methyl, ethyl, n-propyl and/or n-butyl.

The term "ether" as used herein refers to a group of general formula R-O-R' wherein an oxygen atom is connected to two alkyl groups. The term "branched C₃-C₁₀-ether" as used herein refers to an ether group which has 3 to 10 carbon atoms wherein at least one of the alkyl groups R and R' is branched.

The term "fluorinated" alkyl group as used herein refers to alkyl groups wherein at least one carbon-bound hydrogen atom is substituted by fluorine. The term "perfluorinated" alkyl group as used herein refers to alkyl groups wherein all carbon-bound hydrogen atoms are substituted by fluorine.

The substituent R¹ is a fluorinated alkyl or ether group. The substituent R¹ may be a fluorinated linear C₁-C₄-alkyl group or a fluorinated branched C₃-C₄-alkyl group, preferably comprising a -CH₂- moiety. The substituent R¹ particularly may be a fluorinated linear C₁-C₄-alkyl group of the formula CₙF₂ₙ₊₁-(CH₂)ₓ- wherein n is 0, 1, 2 or 3 and x is 1, 2, 3 or 4, with the provision that the sum of n and x is 1, 2, 3 or 4. Such a substituent R¹ would allow for HF to be formed which is suspected to contribute to the passivation process. The substituent R¹ preferably is selected from the group comprising perfluorinated linear C₁-C₄-alkyl and/or perfluorinated branched C₃-C₄-alkyl. The substituent R¹ may be selected from the group comprising perfluorinated methyl, ethyl, n-propyl or n-butyl, or isopropyl, isobutyl, sec-butyl and/or tert-butyl. Preferably, the substituent R¹ may be a linear perfluorinated alkyl group selected from perfluorinated methyl, ethyl, n-propyl and n-butyl.

The substituent R¹ also may be a fluorinated linear C₂-C₄-ether or branched C₃-C₄-ether. Preferably, the substituent R¹ may be a perfluorinated linear C₂-C₄-ether or branched C₃-C₄-ether. A perfluorinated linear C₂-C₄-ether may be selected from the group of CF₃-O-C₃F₆-, C₂F₅-O-C₂F₄- and C₃F₇-O-CF₂-. Particularly C₂F₅-O-C₂F₄- was found to provide in sulfonimides high conductivities.

In embodiments, the substituents R² and R³ may be identical or different from each other, selected from the group comprising a linear C₂-C₅-ether and/or a branched C₃-C₁₀-ether. In a preferred embodiment, the substituents R² and R³ are a linear C₂-C₅-ether. A C₅-ether may be selected from -CH₂-O-C₄H₉, -C₂H₄-O-C₃H₇, -C₃H₆-O-C₂H₅ and/or -C₄H₈-O-CH₃. Preferably, the substituents R² and R³ are a linear C₂-C₄-ether. In a preferred embodiment, the linear C₂-C₄-ether is selected from the group comprising -CH₂-O-CH₃, -CH₂-O-C₂H₅, -C₂H₄-O-CH₃,-C₂H₄-O-C₂H₅, -CH₂-O-C₃H₇, -C₃H₆-O-CH₃. Preferably, the linear C₂-C₄-ether is a C₂- or C₃-ether selected from the group comprising -CH₂-O-CH₃, -CH₂-O-C₂H₅ und/or -C₂H₄-O-CH₃. In other embodiments, R² and R³ are a branched C₄-C₇-ether. Preferred branched C₄-C₇-ethers are selected from the group comprising -CH₂-O-CH(CH₃)₂, -CH(CH₃)-CH₂-O-CH₃, -C₂H₄-O-CH(CH₃)₂, -CH(CH₃)-CH₂-O-CH(CH₃)₂ and/or -CH(CH₃)-CH₂-O-C₂H₅.

In a preferred embodiment, the fluorinated sulfonamide is 1,1,2,2,3,3,4,4,4-nonafluoro-N,N-bis(2-methoxyethyl)butane-1-sulfonamide. The corresponding chemical formula of 1,1,2,2,3,3,4,4,4-nonafluoro-N,N-bis(2-methoxyethyl)butane-1-sulfonamide is given below:

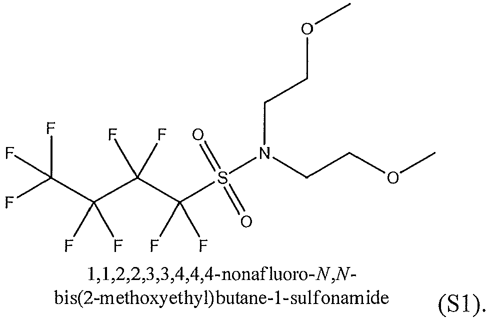

Another aspect of the invention relates to an electrolyte solution comprising an electrolyte salt and an electrolyte solvent, wherein the electrolyte solvent comprises at least one fluorinated sulfonamide according to the general formula (1) R¹-SO₂-NR²R³ wherein:
- R¹: is selected from the group comprising fluorinated linear C₁-C₄-alkyl, branched C₃-C₄-alkyl, linear C₂-C₄-ether and/or branched C₃-C₄-ether;
- R², R³: are selected, the same or independently of the other, from the group comprising linear C₂-C₅-ether and/or branched C₃-C₁₀-ether.

It was found that the detrimental anodic aluminum dissolution in organic solvent-based electrolytes comprising sulfonamide-based lithium salts was suppressed by using fluorinated sulfonamide-based electrolyte solvents. This advantageously results in an enhanced safety of the electrolyte, and thus of the overall battery.

The substituent R¹ may be a fluorinated linear C₁-C₄-alkyl group or a fluorinated branched C₃-C₄-alkyl group, preferably comprising a -CH₂- moiety. The substituent R¹ particularly may be a fluorinated linear C₁-C₄-alkyl group of the formula CₙF₂ₙ₊₁-(CH₂)ₓ- wherein n is 0, 1, 2 or 3 and x is 1, 2, 3 or 4, with the provision that the sum of n and x is 1, 2, 3 or 4. Such a substituent R¹ would allow for HF to be formed which is suspected to contribute to the passivation process. The substituent R¹ preferably is selected from the group comprising perfluorinated linear C₁-C₄-alkyl and/or perfluorinated branched C₃-C₄-alkyl.

The beneficial effect of passivating the aluminum current collector was particularly observed for sulfonamides wherein the substituent R¹ was a perfluorinated alkyl group. The substituent R¹ may be selected from the group comprising perfluorinated methyl, ethyl, n-propyl or n-butyl, or isopropyl, isobutyl, sec-butyl and/or tert-butyl. Preferably, the substituent R¹ may be a linear perfluorinated alkyl group selected from perfluorinated methyl, ethyl, n-propyl and n-butyl. Good results in the prevention of anodic aluminum dissolution and prevention of corrosion of aluminum current collectors were observed in LiTFSI-containing electrolytes using a sulfonamide with R¹ being C₄F₉.

The substituent R¹ also may be a fluorinated linear C₂-C₄-ether or branched C₃-C₄-ether. Preferably, the substituent R¹ may be a perfluorinated linear C₂-C₄-ether or branched C₃-C₄-ether. A perfluorinated linear C₂-C₄-ether may be selected from the group of CF₃-O-C₃F₆-, C₂F₅-O-C₂F₄- and C₃F₇-O-CF₂-. Particularly C₂F₅-O-C₂F₄- was found to provide in sulfonimides high conductivities.

In embodiments, the substituents R² and R³ may be identical or different from each other, selected from the group comprising a linear C₂-C₅-ether and/or a branched C₃-C₁₀-ether. Good results in the suppression of anodic aluminum dissolution and corrosion of aluminum current collectors was observed in LiTFSI-containing electrolytes using sulfonamides with R² and R³ being ether substituents.

In a preferred embodiment, the substituents R² and R³ are identical or different from each other a linear C₂-C₅-ether. A linear C₅-ether may be selected from -CH₂-O-C₄H₉, -C₂H₄-O-C₃H₇, -C₃H₆-O-C₂H₅ and/or -C₄H₈-O-CH₃. Preferably, the substituents R² and R³ are identical or different from each other a linear C₂-C₄-ether. In a preferred embodiment, the linear C₂-C₄-ether is selected from the group comprising -CH₂-O-CH₃, -CH₂-O-C₂H₅, -C₂H₄-O-CH₃, - C₂H₄-O-C₂H₅, -CH₂-O-C₃H₇, -C₃H₆-O-CH₃. Preferably, the linear C₂-C₄-ether is a C₂- or C₃-ether selected from the group comprising -CH₂-O-CH₃, -CH₂-O-C₂H₅ und/or -C₂H₄-O-CH₃. Advantageously, smaller ether groups provide lower viscosity and hence support a higher conductivity. In other embodiments, R² and R³ are a branched C₄-C₇-ether. It is preferred that the oxygen atom is not shielded by directly adjacent alkyl branches. Preferred branched C₄-C₇-ethers are selected from the group comprising -CH₂-O-CH(CH₃)₂, -CH(CH₃)-CH₂-O-CH₃, -C₂H₄-O-CH(CH₃)₂, -CH(CH₃)-CH₂-O-CH(CH₃)₂ and/or -CH(CH₃)-CH₂-O-C₂H₅.

In a preferred embodiment, the fluorinated sulfonamide is 1,1,2,2,3,3,4,4,4-nonafluoro-N,N-bis(2-methoxyethyl)butane-1-sulfonamide. It could be shown that particularly 1,1,2,2,3,3,4,4,4-nonafluoro-N,N-bis(2-methoxyethyl)butane-1-sulfonamide as electrolyte solvent for electrolytes containing LiTFSI as the electrolyte salt resulted in the prevention of anodic aluminum dissolution, and no corrosion was observed for an aluminum current collector, even after 100 potentiodynamic cyclic sweeps.

The electrolyte solution comprises at least one fluorinated sulfonamide according to the general formula (1). In embodiments, the electrolyte solution may comprise two or more or a mixture of the fluorinated sulfonamides.

The fluorinated sulfonamide is usable for the suppression of aluminum dissolution in an electrochemical energy storage device particularly an alkali or alkaline earth metal-based battery containing sulfonimides or other electrolyte salts suffering aluminum current collector corrosion. The fluorinated sulfonamide may be used as only electrolyte solvent for lithium sulfonimide-based electrolytes.

Alternatively, the fluorinated sulfonamide may be used as electrolyte co-solvent for lithium sulfonimide-based electrolytes. In embodiments, the electrolyte solution comprises the fluorinated sulfonamide in a range from ≥ 75 wt% to ≤ 100 wt%, preferably in a range of ≥ 85 wt% to ≤ 100 wt%, more preferably in a range of ≥ 90 wt% to ≤ 100 wt%, referring to a total amount of the electrolyte solvent of 100 wt%. Advantageously, an amount of ≥ 75 wt.-% of fluorinated sulfonamide in standard non-fluorinated carbonates significantly can suppress aluminum current collector corrosion. If not indicated otherwise, the ratios of electrolyte solvents or compounds as given refer to a respective weight ratio. Weight percent, abbreviated wt% or wt.-% are synonyms that refer to the concentration of a compound as the weight of the compound divided by the weight of the composition and multiplied by 100. The total amount of all solvents of the electrolyte solution does not exceed 100 wt%.

The fluorinated sulfonamides are usable for the suppression of aluminum dissolution of a current collector as co-solvent with non-fluorinated electrolyte solvents such as carbonates, esters, lactones or nitriles, in particular with non-fluorinated organic carbonates. This is of particular benefit as non-fluorinated organic carbonates are used as standard solvents in commercial lithium-ion batteries. In embodiments, the electrolyte solution comprises at least one non-fluorinated organic carbonate selected from the group comprising ethylene carbonate, ethyl methyl carbonate, propylene carbonate, dimethyl carbonate, diethyl carbonate, and mixtures thereof. A preferred mixture of carbonate solvents is a carbonate solvent mixture of ethylene carbonate (EC) and dimethyl carbonate (DMC), for example in a weight ratio of 1:1.

The use of fluorinated sulfonamides as electrolyte (co-)solvent enables the utilization of sulfonimide-based conductive salts, for example in lithium-ion batteries. In embodiments, the electrolyte salt is selected from the group comprising alkali or alkaline earth metal sulfonimide, methide, sulfonate or oxalatoborate salts. The alkali or alkaline earth metal salt preferably is selected from lithium, sodium, potassium, magnesium, or calcium salts. The electrolyte salt particularly is a lithium salt. In other embodiments, Al or Zn sulfonimide, methide, sulfonate and oxalatoborate salts may be usable conductive salts.

In a preferred embodiment, the alkali or alkaline earth metal sulfonimide or sulfonate salt is a lithium salt selected from the group comprising lithium bis(trifluoromethanesulfonyl)imide LiN(SO₂CF₃)₂ (LiTFSI), lithium bis(fluorosulfonyl)imide LiN(FSO₂)₂ (LiFSI), lithium (trifluoromethylsulfonyl)(nonafluorobutanesulfonyl)imide LiN(SO₂CF₃)(SO₂C₄F₉), lithium (fluorosulfonyl)(nonafluorobutanesulfonyl)imide LiN(SO₂F)(SO₂C₄F₉), lithium (nonafluoro butan-2-one sulfonyl)(trifluoromethylsulfonyl)imide LiN(SO₂C₂F₄OC₂F₅)(SO₂CF₃), lithium (nonafluoro butan-2-one sulfonyl)(fluorosulfonyl)imide LiN(SO₂C₂F₄OC₂F₅)(SO₂F) and/or lithium trifluoromethanesulfonate Li(CF₃)SO₃ (LiTf). Preferred lithium sulfonimide salts are bis(trifluoromethanesulfonyl)imide (LiTFSI) and lithium bis(fluorosulfonyl)imide (LiFSI). A preferred methide salt is LiC(CF₃SO₂)₃. A preferred oxalatoborate salt is lithium bis(oxalato)borate (LiBOB).

Sulfonimide salts provide a high ionic conductivity and show enhanced thermal and electrochemical stability. Further, sulfonimide salts prevent an immediate formation of HF by hydrolysis as may occur with LiPF6. Anodic aluminum dissolution, which is associated with a use of sulfonimide salts and usually is referred to as "corrosion", can be prevented by using a fluorinated sulfonamide according to the invention. A use of sulfonimide salts such as LiTFSI will result in significantly safer lithium-ion batteries. Particularly LiTFSI, LiFSI and the sulfonate Li-Triflate, which are known to suffer anodic aluminum dissolution, can advantageously be utilized as electrolyte salts in lithium-ion batteries using a fluorinated sulfonamide as the solvent.

Further electrolyte salts may be selected from the group comprising alkali or alkaline earth metal, Al or Zn salts of CF3- and CN-substituted Hückel-anions, of secondary carbanions substituted with (CF₃SO₂)₂CH⁻, of tertiary carbanions substituted with (CF₃SO₂)₃C⁻-, (N≡C)₃C⁻- or (FSO₂)₃C⁻-, pentacyano cyclopentadienyl, arylsulfonyl, trifluoromethane sulfonyl amide anions, SO₃⁻- end-capped polyethylene glycol, or sulfonamide end-capped polyethylene glycol, and mixtures of these salts.

A preferred -CF₃ and -CN-substituted Hückel-anion is 4,5-dicyano-2-(trifluoromethyl)imidazolide. Other anions of electrolyte salts are secondary carbanions substituted by electron-withdrawing groups such as (CF₃SO₂)₂CH⁻ and tertiary carbanions substituted by electron-withdrawing groups such as (CF₃SO₂)3C⁻ which is also denoted methide, (N≡C)₃C⁻ or (FSO₂)₃C⁻, and aromatic anions such as pentacyano cyclopentadienyl (N≡C)₅C₅⁻. The term "arylsulfonyl, trifluoromethane sulfonyl amide anion" according to the invention is to be understood as meaning an anion (X-C₆H₄-SO₂)(CₙF₂ₙ₊₁SO₂)N⁻ wherein X is selected from the group of electron-donating substituents methyl, methoxy and/or amine or the group of electron-withdrawing groups nitrile, nitro and/or CF₃SO₂, or X is a functionality allowing its polymerization such as a double bond (vinyl) or an epoxy group. The term "SO₃⁻-end-capped polyethylene glycol" according to the invention is to be understood as meaning monomethyl PEG end-capped by one or two ionic moieties according to the formula (6) CH3-(O-CH₂-CH₂)ₙ-O-SO₃⁻ wherein 1 ≤ n ≤ 23 and preferably 3 ≤ n ≤ 8 and, alternatively, PEG twice end-capped by SO₃⁻ according to the formula (7) "O₃S-(O-CH₂-CH₂)ₙ-O-SO₃⁻ wherein 1 ≤ n ≤ 46 and preferably 8 ≤ n ≤ 20. The electrolyte solvent may be a liquid or an ionic liquid, for example based on the anions above. The electrolyte can fill a porous separator or it can be a gelled electrolyte as a copolymer poly(vinylidene fluoride-co-hexafluoropropene) (PVDF-HFP) or a polymethacrylonitrile network.

Another aspect of the invention refers to an electrochemical energy storage device comprising an electrolyte solution according to the invention. The electrochemical energy storage device preferably is an alkali or alkaline earth metal-based or an Al-based or Zn-based electrochemical energy storage device. The electrochemical energy storage device particularly is a lithium battery, a primary or secondary lithium-ion battery or lithium polymer battery or a lithium-ion capacitor. The term "energy storage device" comprises primary and secondary, i.e., rechargeable batteries. However, rechargeable batteries or accumulators are also denoted with the term "battery" which usually is used as a generic term. Hence, for simplicity if not denoted otherwise, in the present invention the term "battery" is used synonymous to also designate "accumulators". Preferably, the electrolyte solution according to the invention is usable for a primary or secondary lithium-ion battery or a lithium polymer battery. A lithium-ion battery for example comprises a first electrode of a cathodic material, a second electrode of an anodic material and an electrolyte. Advantageously, an electrochemical energy storage device, particularly a secondary lithium battery, comprising the electrolyte solution according to the invention provides increased safety.

The electrolyte solution of the electrochemical energy storage device comprises an electrolyte salt and an electrolyte solvent, wherein the electrolyte solvent comprises at least one fluorinated sulfonamide according to the general formula (1) R¹-SO₂-NR²R³ wherein:
- R¹: is selected from the group comprising fluorinated linear C₁-C₄-alkyl, branched C₃-C₄-alkyl, linear C₂-C₄-ether and/or branched C₃-C₄-ether;
- R², R³: are selected, the same or independently of the other, from the group comprising linear C₂-C₅-ether and/or branched C₃-C₁₀-ether.

The substituent R¹ may be a fluorinated linear C₁-C₄-alkyl group or a fluorinated branched C₃-C₄-alkyl group, preferably comprising a -CH₂- moiety. The substituent R¹ particularly may be a fluorinated linear C₁-C₄-alkyl group of the formula CₙF₂ₙ₊₁-(CH₂)ₓ- wherein n is 0, 1, 2 or 3 and x is 1, 2, 3 or 4, with the provision that the sum of n and x is 1, 2, 3 or 4. Such a substituent R¹ would allow for HF to be formed which is suspected to contribute to the passivation process. The substituent R¹ preferably is selected from the group comprising perfluorinated linear C₁-C₄-alkyl and/or perfluorinated branched C₃-C₄-alkyl. The substituent R¹ may be selected from the group comprising perfluorinated methyl, ethyl, n-propyl or n-butyl, or isopropyl, isobutyl, sec-butyl and/or tert-butyl. Preferably, the substituent R¹ may be a linear perfluorinated alkyl group selected from perfluorinated methyl, ethyl, n-propyl and n-butyl.

The substituent R¹ also may be a fluorinated linear C₂-C₄-ether or branched C₃-C₄-ether. Preferably, the substituent R¹ may be a perfluorinated linear C₂-C₄-ether or branched C₃-C₄-ether. A perfluorinated linear C₂-C₄-ether may be selected from the group of CF₃-O-C₃F₆-, C₂F₅-O-C₂F₄- and C₃F₇-O-CF₂-. Particularly C₂F₅-O-C₂F₄- was found to provide in sulfonimides high conductivities.

In embodiments, the substituents R² and R³ may be identical or different from each other, selected from the group comprising a linear C₂-C₅-ether and/or a branched C₃-C₁₀-ether. In a preferred embodiment, the substituents R² and R³ are a linear C₂-C₅-ether. A C₅-ether may be selected from -CH₂-O-C₄H₉, -C₂H₄-O-C₃H₇, -C₃H₆-O-C₂H₅ and/or -C₄H₈-O-CH₃. Preferably, the substituents R² and R³ are a linear C₂-C₄-ether. In a preferred embodiment, the linear C₂-C₄-ether is selected from the group comprising -CH₂-O-CH₃, -CH₂-O-C₂H₅, -C₂H₄-O-CH₃, - C₂H₄-O-C₂H₅, -CH₂-O-C₃H₇, -C₃H₆-O-CH₃. Preferably, the linear C₂-C₄-ether is a C₂- or C₃-ether selected from the group comprising -CH₂-O-CH₃, -CH₂-O-C₂H₅ und/or -C₂H₄-O-CH₃. In other embodiments, R² and R³ are a branched C₄-C₇-ether. Preferred branched C₄-C₇-ethers are selected from the group comprising -CH₂-O-CH(CH₃)₂, -CH(CH₃)-CH₂-O-CH₃, -C₂H₄-O-CH(CH₃)₂, -CH(CH₃)-CH₂-O-CH(CH₃)₂ and/or -CH(CH₃)-CH₂-O-C₂H₅. In a preferred embodiment, the fluorinated sulfonamide is 1,1,2,2,3,3,4,4,4-nonafluoro-N,N-bis(2-methoxyethyl)butane-1-sulfonamide.

In embodiments, the electrolyte solution comprises the fluorinated sulfonamide in a range from ≥ 75 wt% to ≤ 100 wt%, preferably in a range of ≥ 85 wt% to ≤ 100 wt%, more preferably in a range of ≥ 90 wt% to ≤ 100 wt%, referring to a total amount of the electrolyte solvent of 100 wt%.

The fluorinated sulfonamides are usable for the suppression of aluminum dissolution of a current collector as co-solvent with non-fluorinated electrolyte solvents such as carbonates, esters, lactones or nitriles, in particular with non-fluorinated organic carbonates. In embodiments, the electrolyte solution comprises at least one non-fluorinated organic carbonate selected from the group comprising ethylene carbonate, ethyl methyl carbonate, propylene carbonate, dimethyl carbonate, diethyl carbonate, and mixtures thereof. A preferred mixture of carbonate solvents is a carbonate solvent mixture of ethylene carbonate (EC) and dimethyl carbonate (DMC), for example in a weight ratio of 1:1.

In embodiments, the electrolyte salt is selected from the group comprising alkali or alkaline earth metal sulfonimide, methide, sulfonate or oxalatoborate salts. The alkali or alkaline earth metal salt preferably is selected from lithium, sodium, potassium, magnesium, or calcium salts. The electrolyte salt particularly is a lithium salt. In other embodiments, Al or Zn sulfonimide, methide, sulfonate and oxalatoborate salts may be usable conductive salts. In a preferred embodiment, the alkali or alkaline earth metal sulfonimide or sulfonate salt is a lithium salt selected from the group comprising lithium bis(trifluoromethanesulfonyl)imide LiN(SO₂CF₃)₂ (LiTFSI), lithium bis(fluorosulfonyl)imide LiN(FSO₂)₂ (LiFSI), lithium (trifluoromethylsulfonyl)(nonafluorobutanesulfonyl)imide LiN(SO₂CF₃)(SO₂C₄F₉), lithium (fluorosulfonyl)(nonafluorobutanesulfonyl)imide LiN(SO₂F)(SO₂C₄F₉), lithium (nonafluoro butan-2-one sulfonyl)(trifluoromethylsulfonyl)imide LiN(SO₂C₂F₄OC₂F₅)(SO₂CF₃), lithium (nonafluoro butan-2-one sulfonyl)(fluorosulfonyl)imide LiN(SO₂C₂F₄OC₂F₅)(SO₂F) and/or lithium trifluoromethanesulfonate Li(CF₃)SO₃ (LiTf). Preferred lithium sulfonimide salts are bis(trifluoromethanesulfonyl)imide (LiTFSI) and lithium bis(fluorosulfonyl)imide (LiFSI). A preferred methide salt is LiC(CF₃SO₂)₃. A preferred oxalatoborate salt is lithium bis(oxalato)borate (LiBOB).

Another aspect of the invention relates to a method for preventing aluminum current collector corrosion in an electrochemical energy storage device, the method comprising the step of using an electrolyte solution comprising an electrolyte salt and an electrolyte solvent, wherein the electrolyte solvent comprises at least one fluorinated sulfonamide according to the general formula (1) R¹-SO₂-NR²R³ wherein:
- R¹: is selected from the group comprising fluorinated linear C₁-C₄-alkyl, branched C₃-C₄-alkyl, linear C₂-C₄-ether and/or branched C₃-C₄-ether;
- R², R³: are selected, the same or independently of the other, from the group comprising linear C₂-C₅-ether and/or branched C₃-C₁₀-ether.

Advantageously, the method increases the safety of an electrochemical energy storage device, particularly a lithium battery, a primary or secondary lithium-ion battery or lithium polymer battery or a lithium-ion capacitor. Preferably, the device is a primary or secondary lithium-ion battery or a lithium polymer battery.

Another aspect of the invention refers to the use of a fluorinated sulfonamide according to the general formula (1) R¹-SO₂-NR²R³ wherein:
- R¹: is selected from the group comprising fluorinated linear C₁-C₄-alkyl, branched C₃-C₄-alkyl, linear C₂-C₄-ether and/or branched C₃-C₄-ether;
- R², R³: are selected, the same or independently of the other, from the group comprising linear C₂-C₅-ether and/or branched C₃-C₁₀-ether,
for the prevention of aluminum current collector corrosion in an electrochemical energy storage device.

Referring to the method for preventing aluminum current collector corrosion in an electrochemical energy storage device and/or to the use of the fluorinated sulfonamide for the prevention of aluminum current collector corrosion, the substituent R¹ is a fluorinated alkyl or ether group. The substituent R¹ may be a fluorinated linear C₁-C₄-alkyl group or a fluorinated branched C₃-C₄-alkyl group, preferably comprising a -CH₂- moiety. The substituent R¹ particularly may be a fluorinated linear C₁-C₄-alkyl group of the formula CₙF₂ₙ₊₁-(CH₂)ₓ-wherein n is 0, 1, 2 or 3 and x is 1, 2, 3 or 4, with the provision that the sum of n and x is 1, 2, 3 or 4. Such a substituent R¹ would allow for HF to be formed which is suspected to contribute to the passivation process. The substituent R¹ preferably is selected from the group comprising perfluorinated linear C₁-C₄-alkyl and/or perfluorinated branched C₃-C₄-alkyl. Preferably, the substituent R¹ may be selected from the group comprising perfluorinated methyl, ethyl, n-propyl or n-butyl, or isopropyl, isobutyl, sec-butyl and/or tert-butyl. Preferably, the substituent R¹ may be a linear perfluorinated alkyl group selected from perfluorinated methyl, ethyl, n-propyl and n-butyl. The substituent R¹ also may be a fluorinated linear C₂-C₄-ether or branched C₃-C₄-ether. Preferably, the substituent R¹ may be a perfluorinated linear C₂-C₄-ether or branched C₃-C₄-ether. A perfluorinated linear C₂-C₄-ether may be selected from the group of CF₃-O-C₃F₆-, C₂F₅-O-C₂F₄- and C₃F₇-O-CF₂-.

In embodiments of the method or the use, the substituents R² and R³ may be identical or different from each other, selected from the group comprising a linear C₂-C₅-ether and/or a branched C₃-C₁₀-ether. In a preferred embodiment, the substituents R² and R³ are a linear C₂-C₅-ether. A C₅-ether may be selected from -CH₂-O-C₄H₉, -C₂H₄-O-C₃H₇, -C₃H₆-O-C₂H₅ and/or -C₄H₈-O-CH₃. Preferably, the substituents R² and R³ are a linear C₂-C₄-ether. In a preferred embodiment, the linear C₂-C₄-ether is selected from the group comprising -CH₂-O-CH₃, -CH₂-O-C₂H₅, -C₂H₄-O-CH₃, -C₂H₄-O-C₂H₅, -CH₂-O-C₃H₇, -C₃H₆-O-CH₃. Preferably, the linear C₂-C₄-ether is a C₂- or C₃-ether selected from the group comprising -CH₂-O-CH₃, - CH₂-O-C₂H₅ und/or -C₂H₄-O-CH₃. In other embodiments, R² and R³ are a branched C₄-C₇-ether. Preferred branched C₄-C₇-ethers are selected from the group comprising -CH₂-O-CH(CH₃)₂, -CH(CH₃)-CH₂-O-CH₃, -C₂H₄-O-CH(CH₃)₂, -CH(CH₃)-CH₂-O-CH(CH₃)₂ and/or -CH(CH₃)-CH₂-O-C₂H₅. In a preferred embodiment, the fluorinated sulfonamide is 1,1,2,2,3,3,4,4,4-nonafluoro-N,N-bis(2-methoxyethyl)butane-1-sulfonamide.

In embodiments, the electrolyte solution comprises the fluorinated sulfonamide in a range from ≥ 75 wt% to ≤ 100 wt%, preferably in a range of ≥ 85 wt% to ≤ 100 wt%, more preferably in a range of ≥ 90 wt% to ≤ 100 wt%, referring to a total amount of the electrolyte solvent of 100 wt%. The fluorinated sulfonamides are usable for the suppression of aluminum dissolution of a current collector as co-solvent with non-fluorinated electrolyte solvents such as carbonates, esters, lactones or nitriles, in particular with non-fluorinated organic carbonates. In embodiments, the electrolyte solution comprises at least one non-fluorinated organic carbonate selected from the group comprising ethylene carbonate, ethyl methyl carbonate, propylene carbonate, dimethyl carbonate, diethyl carbonate, and mixtures thereof. A preferred mixture of carbonate solvents is a carbonate solvent mixture of ethylene carbonate (EC) and dimethyl carbonate (DMC), for example in a weight ratio of 1:1.

In embodiments, the electrolyte salt is selected from the group comprising alkali or alkaline earth metal sulfonimide, methide, sulfonate or oxalatoborate salts. The alkali or alkaline earth metal salt preferably is selected from lithium, sodium, potassium, magnesium, or calcium salts. The electrolyte salt particularly is a lithium salt. In other embodiments, Al or Zn sulfonimide, methide, sulfonate and oxalatoborate salts may be usable conductive salts. In a preferred embodiment, the alkali or alkaline earth metal sulfonimide or sulfonate salt is a lithium salt selected from the group comprising lithium bis(trifluoromethanesulfonyl)imide LiN(SO₂CF₃)₂ (LiTFSI), lithium bis(fluorosulfonyl)imide LiN(FSO₂)₂ (LiFSI), lithium (trifluoromethylsulfonyl)(nonafluorobutanesulfonyl)imide LiN(SO₂CF₃)(SO₂C₄F₉), lithium (fluorosulfonyl)(nonafluorobutanesulfonyl)imide LiN(SO₂F)(SO₂C₄F₉), lithium (nonafluoro butan-2-one sulfonyl)(trifluoromethylsulfonyl)imide LiN(SO₂C₂F₄OC₂F₅)(SO₂CF₃), lithium (nonafluoro butan-2-one sulfonyl)(fluorosulfonyl)imide LiN(SO₂C₂F₄OC₂F₅)(SO₂F) and/or lithium trifluoromethanesulfonate Li(CF₃)SO₃ (LiTf). Preferred lithium sulfonimide salts are bis(trifluoromethanesulfonyl)imide (LiTFSI) and lithium bis(fluorosulfonyl)imide (LiFSI). A preferred methide salt is LiC(CF₃SO₂)₃. A preferred oxalatoborate salt is lithium bis(oxalato)borate (LiBOB).

Unless otherwise defined, the technical and scientific terms used herein have the same meaning as commonly understood by one of ordinary skill in the art to which this invention belongs.

The examples which follow serve to illustrate the invention in more detail but do not constitute a limitation thereof.

In the figures show:
- Figure 1: Ionic conductivities of solutions comprising 1M LiTFSI in 1,1,2,2,3,3,4,4,4-nonafluoro-N,N-bis(2-methoxyethyl)butane-1-sulfonamide (S1), EC/DMC (1:1), and S1/EC/DMC (2:1:1).
- Figure 2: a cyclic voltammogram of a 1M solution of LiTFSI in 1,1,2,2,3,3,4,4,4-nonafluoro-N,N-bis(2-methoxyethyl)butane-1-sulfonamide (S1) for 100 cycles using aluminum as working electrode.
- Figure 3: a cyclic voltammogram of a 0.5M solution of LiTFSI in 1,1,2,2,3,3,4,4,4-nonafluoro-N,N-bis(2-methoxyethyl)butane-1-sulfonamide (S1) for 100 cycles using aluminum as working electrode.
- Figure 4: a cyclic voltammogram of a 1M solution of LiTFSI in a solvent mixture of 33 wt% of 1,1,2,2,3,3,4,4,4-nonafluoro-N,N-bis(2-methoxyethyl)butane-1-sulfonamide (S1) in EC/DMC (1:1:1) for 100 cycles using aluminum as working electrode.
- Figure 5: a cyclic voltammogram of a 1M solution of LiTFSI in a solvent mixture of 50 wt% of 1,1,2,2,3,3,4,4,4-nonafluoro-N,N-bis(2-methoxyethyl)butane-1-sulfonamide (S1) in EC/DMC (2:1:1) for 100 cycles using aluminum as working electrode.
- Figure 6: a cyclic voltammogram of a 1M solution of LiTFSI in a solvent mixture of 66 wt% of 1,1,2,2,3,3,4,4,4-nonafluoro-N,N-bis(2-methoxyethyl)butane-1-sulfonamide (S1) in EC/DMC (4:1:1) for 100 cycles using aluminum as working electrode.

### Example 1

### Synthesis of 1,1,2,2,3,3,4,4,4-nonafluoro-N,N-bis(2-methoxyethyl)butane-1-sulfonamide (S1)

A solution of 1 eq. of Bis(2-methoxyethyl)amine (>98%, Sigma-Aldrich) in freshly distilled tetrahydrofuran was cooled at -70 °C in a round bottom flask. To this solution, a 2.5 M solution of n-butyllithium (1eq.) in hexane was added. Subsequently, a solution of perfluorobutane sulfonylfluoride in tetrahydrofuran was added dropwise while the flask was gradually warmed up to room temperature (25 ± 2 °C). The reaction was continued overnight. Afterwards, the system was washed with water and the organic phases were extracted with a solution of water saturated with sodium carbonate.

The organic phases were dried under magnesium sulfate. The sulfonamide was obtained as a yellow liquid by distillation under reduced pressure at 100 mmHg and room temperature. The total yield was 60-65%.

The physico-chemical properties of 1,1,2,2,3,3,4,4,4-nonafluoro-N,N-bis(2-methoxyethyl)butane-1-sulfonamide are summarised in table 1 below.

The density of the solvents was determined by weighing 1 ml of solvent, previously stabilized at 25°C during 1 hour, with a Mettler Toledo XS analytical balance. The measurement was repeated 10 times to ensure reproducibility.

The boiling point, T_{B}, was determined using a distillation apparatus by controlling the temperature of the pure solvent vapor. The measurements were repeated at least 3 times to ensure reproducibility. The melting point, T_{M}, was determined by Differential Scanning Calorimetry (DSC), using a TA Instrument DSC 2920 CE and applying a heat rate of 2 °C min⁻¹.

Viscosity measurements were carried out by means of a TA Instrument DHR-3 cone-plate rheometer using a stainless steel cone of 60 mm in diameter and an angle α of 0°59'42". To avoid any water contamination during the measurement, the device was shielded by a plastic glove box, kept under dry air. The shear rate was fixed at 10 s⁻¹, after ensuring the Newtonian behavior of each sample. The measurements were carried out in a range of 10 to 60 °C. The measurement was repeated 3-times to ensure reproducibility; uncertainties of viscosity values are estimated to be less than 5% of the determined values.

The dielectric constants was determined by electrochemical impedance spectroscopy (EIS), using a Solartron 2100A ModuLab in the frequency range 5 Hz - 1 MHz. The samples were placed in a home-made dip-type glass cell with two plate isolated electrodes, which were maintained at an electrical potential difference of 5 V during the frequency sweep.

**Table 1**

| 1,1,2,2,3,3,4,4,4-nonafluoro-N,N-bis(2-methoxyethyl)butane-1-sulfonamide (S1) | | | |
|---|---|---|---|
| T_{M} / T_{B} [°C] | Density d [g cm⁻³] | Dielectric constant εᵣ at 20 °C | Viscosity η at 20 °C (* 10⁻³ Pa s) |
| -18.5 / >200 | 1.453 ± 0.006 | 16.104 ± 0.037 | 12.120 ± 0.368 |

### Example 2

### Determination of the ionic conductivity of solutions of 1M LiTFSI in 1,1,2,2,3,3,4,4,4-nonafluoro-N,N-bis(2-methoxyethyl)butane-1-sulfonamide (S1)

Ionic conductivities were determined by EIS using an HP 4192A Impedance Analyser in the frequency range 5 Hz to 1MHz. The samples were placed in a dip-type glass cell with Pt electrodes fixed at a constant distance and measurements were performed under argon in a range of -30 °C to 60 °C. The temperature was equilibrated for one hour prior to each measurement. The uncertainties of conductivity values are estimated to be ± 0.3 mS cm⁻¹.

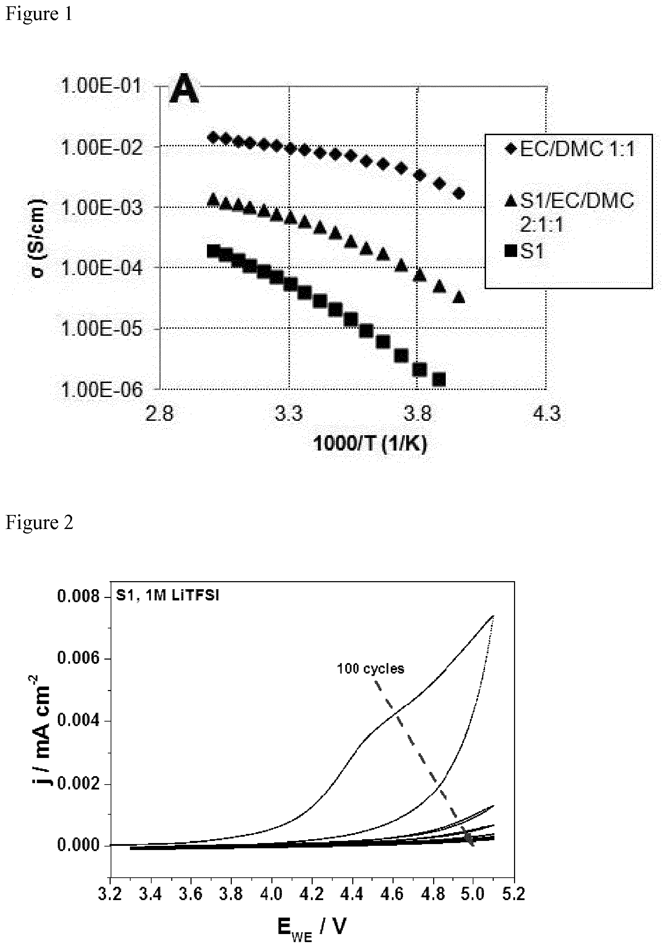

Figure 1 shows the ionic conductivities of solutions comprising 1M LiTFSI in 1,1,2,2,3,3,4,4,4-nonafluoro-N,N-bis(2-methoxyethyl)butane-1-sulfonamide (S1) as the only solvent and in a solvent mixture of 50 wt% S1 in ethylene carbonate and dimethyl carbonate (S1/EC/DMC 2:1:1). An electrolyte solution of 1M LiTFSI in the state-of-the-art carbonate solvent mixture of ethylene carbonate and dimethylcarbonate in a weight ratio of 1:1 (EC/DMC 1:1) was used for comparison.

The conductivity of the solution comprising only S1 was measured to be 4.4 x 10⁻⁵ S/cm, which is lower than the conductivity of the EC/DMC mixture (9.5 x 10⁻³ S/cm). This difference is related presumably to the different viscosity of the mixtures of 143.11 mPa.s *vs* 3.39 mPa.s at 20°C and to the different solvation ability of the molecules. The addition of 50% mol of EC/DMC mixture to S1 permits to increase the conductivity to a value of 6.9 x 10⁻⁴ S/cm, much more acceptable with regard of the battery application.

### Electrochemical characterization

### Solvents and electrolyte compositions for electrochemical studies:

The electrolyte solvent 1,1,2,2,3,3,4,4,4-nonafluoro-*N,N*-bis(2-methoxyethyl)butane-1-sulfonamide (S1) was used as synthesized according to example 1.

Ethylene carbonate (EC) and dimethyl carbonate (DMC), both battery grade, were purchased from UBE and Ferro Corporation.

The concentration of the conducting salt lithium bis(trifluoromethanesulfonyl)imide (LiTFSI, 3M) was set to 0.5 mol L⁻¹ or 1 m L⁻¹. Electrolyte solutions were prepared by mixing the solvent in their respective weight ratios and dissolving LiTFSI in an appropriate amount to yield the desired concentration.

### Example 3

### Determination of anodic aluminum dissolution using 1M LiTFSI in 1,1,2,2,3,3,4,4,4-nonafluoro-N,N-bis(2-methoxyethyl)butane-1-sulfonamide (S1)

An electrolyte solution of 1M LiTFSI in 1,1,2,2,3,3,4,4,4-nonafluoro-*N,N*-bis(2-methoxyethyl)butane-1-sulfonamide (S1) was prepared and cyclic voltammetry was performed by means of a VMP potentiostat/galvanostat (Biologic), performing cyclic voltammetry using an aluminum disk of a diameter of 12 mm (Evonik, 99.99% purity) as working electrode. The sweep rate was set to 5 mV s⁻¹. The reversing potentials were set to 3.3 and 5.1 V vs. Li/Li⁺ and the cell was polarized for 100 continuous cyclic sweeps. Subsequently, the cycled Swagelok® cells were disassembled in an MBraun glove box under argon. The polarized aluminum foils were rinsed with DMC, dried under vacuum, and transferred to a high resolution scanning electron microscope (HRSEM, ZEISS Auriga® microscope), using a self-designed sample holder to avoid contact to air and moisture.

Figure 2 shows the cyclic voltammogram for 1M LiTFSI in S1 for 100 cycles. As can be taken from the figure 2, after the first cycle the observed current density decreased significantly and almost no current was observed subsequently upon the second and the continuous potentiodynamic sweeps. No signs of anodic aluminum dissolution were detectable by performing the cyclic voltammetry.

Also the subsequent SEM analysis of the aluminum foil polarized by the cyclic voltammetry in S1 comprising 1M LiTFSI did not show any indication of anodic aluminum dissolution. This finding confirmed the prevention of aluminum dissolution by using 1,1,2,2,3,3,4,4,4-nonafluoro-*N,N*-bis(2-methoxyethyl)butane-1-sulfonamide (S1) as electrolyte solvent.

### Example 4

### Determination of anodic aluminum dissolution using 0.5M LiTFSI in 1,1,2,2,3,3,4,4,4-nonafluoro-N,N-bis(2-methoxyethyl)butane-1-sulfonamide (S1)

An electrolyte solution of 0.5M LiTFSI in 1,1,2,2,3,3,4,4,4-nonafluoro-*N,N*-bis(2-methoxyethyl)butane-1-sulfonamide (S1) was prepared and cyclic voltammetry and SEM analysis was performed as described in example 4.

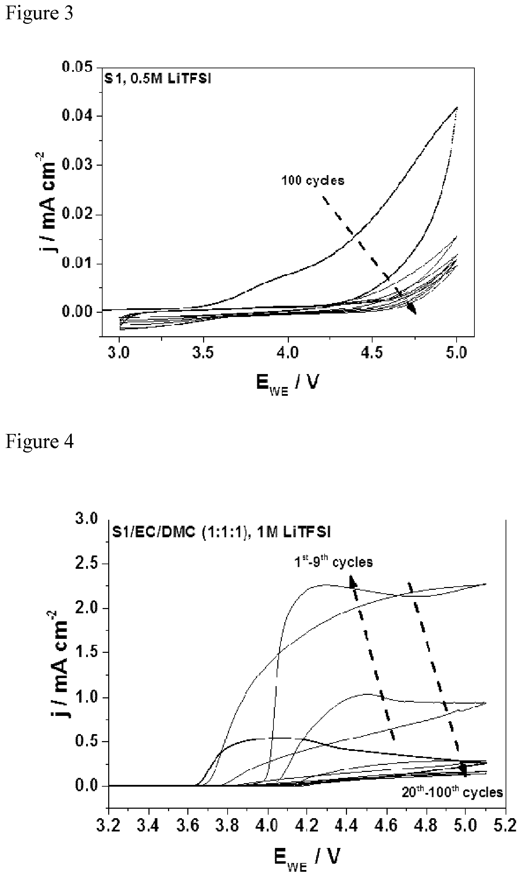

Figure 3 shows the cyclic voltammogram for 0.5M LiTFSI in S1 for 100 cycles. As can be taken from the figure 3, also for a solution of 0.5M LiTFSI in S1 after the first cycle the observed current density decreased significantly and almost no current was observed subsequently upon the second and the continuous potentiodynamic sweeps. No signs of anodic aluminum dissolution were detectable by performing the cyclic voltammetry. Also the subsequent SEM analysis of the aluminum foil polarized by the cyclic voltammetry in S1 comprising 0.5M LiTFSI did not show any indication of anodic aluminum dissolution.

This shows that a 0.5M solution of LiTFSI in S1 inhibits aluminum dissolution and reduces the viscosity of the solution.

### Example 5

### Determination of different amounts of fluorinated sulfonamide as electrolyte solvent

The effect of different amounts of the fluorinated sulfonamide as electrolyte solvent for LiTFSI in regard of the prevention of aluminum current collector corrosion was investigated. Electrolyte solutions of 1M LiTFSI in the state-of-the-art carbonate solvent mixture of ethylene carbonate (EC) and dimethylcarbonate (DMC) in a weight ratio of 1:1 containing different amounts of 1,1,2,2,3,3,4,4,4-nonafluoro-*N,N*-bis(2-methoxyethyl)butane-1-sulfonamide (S1) were prepared. Solutions of 33 wt%, 50 wt% or 66 wt%, respectively, of 1,1,2,2,3,3,4,4,4-nonafluoro-*N,N*-bis(2-methoxyethyl)butane-1-sulfonamide (S1) were prepared by adding S1 in a ratio of 1:1:1, 2:1:1 or 4:1:1, respectively, to a 1:1 mixture of EC and DMC.

Cyclic voltammetry was performed by means of a VMP potentiostat/galvanostat, as described in example 4. For these studies, an aluminum disk of a diameter of 12 mm (Evonik, 99.99% purity) was used as working electrode with lithium metal as counter- and reference-electrode. The sweep rate was set to 5 mV s⁻¹. The reversing potentials were set to 3.3 and 5.1 V vs Li/Li⁺ and the cell was polarized for 100 continuous cyclic sweeps. Subsequently, the cycled cells were disassembled, and the polarized aluminum foils were rinsed, dried and transferred to a high resolution scanning electron microscope.

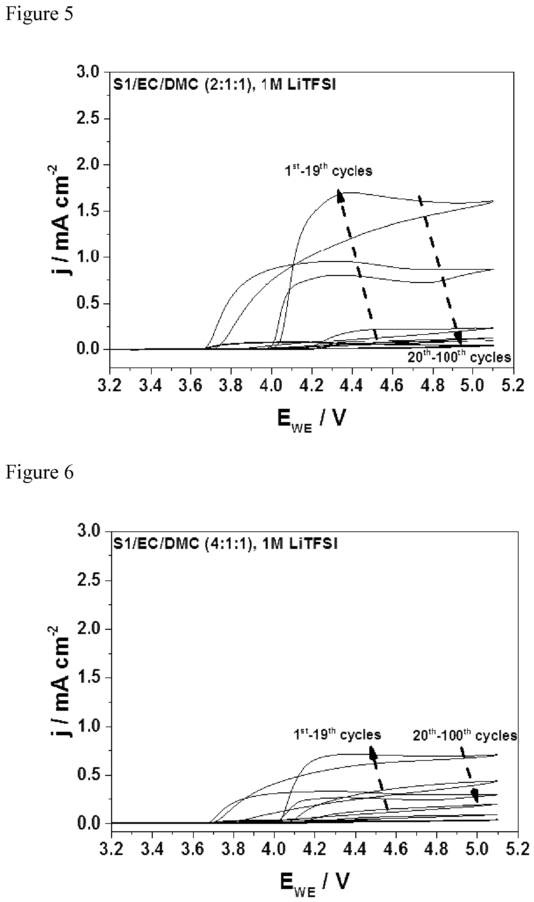

Figures 4, 5 and 6, respectively, show the cyclovoltammograms for 1M LiTFSI in 33 wt%, 50 wt% or 66 wt% of 1,1,2,2,3,3,4,4,4-nonafluoro-*N,N*-bis(2-methoxyethyl)butane-1-sulfonamide (S1) in a 1:1 mixture of EC/DMC for 100 cycles using aluminum as working electrode. Figure 4 shows a continuously increasing evolving current density during the continuous potentiodynamic sweeps. Within the first 9 cyclic sweeps a current density of up to 2.3 mA cm⁻² was detected, indicating a severe anodic dissolution of the aluminum current collector. The subsequent SEM analysis of the aluminum electrode showed a severe corrosion of the aluminum current collector and confirmed the electrochemical findings. The Figure 5 shows that 50 wt% S1 resulted in decreased current densities. However, for the first 19 cycles a current density of about 2 mA cm⁻² was still detected and a severe pitting corrosion at the aluminum surface was observed by subsequent SEM analysis. The cyclic voltammogram given in Figure 6 illustrates further decreased current densities to about 0.75 mA cm⁻² using 66 wt% S1 as electrolyte solvent. An improvement in the suppression of the detrimental aluminum dissolution was confirmed by subsequent SEM analysis. The cyclic voltammograms illustrate that an amount exceeding about 70 wt% of the fluorinated sulfonamide according to the invention should be used to substantially reduce the aluminum current collector corrosion.

## Claims

1. An electrolyte solution comprising an electrolyte salt and an electrolyte solvent, wherein the electrolyte solvent comprises at least one fluorinated sulfonamide according to the general formula (1) R¹-SO₂-NR²R³ wherein:
R¹ is selected from the group comprising fluorinated linear C₁-C₄-alkyl, branched C₃-C₄-alkyl, linear C₂-C₄-ether and/or branched C₃-C₄-ether;
R², R³ are selected, the same or independently of the other, from the group comprising linear C₂-C₅-ether and/or branched C₃-C₁₀-ether.

2. The electrolyte solution according to claim 1, wherein R¹ is selected from the group comprising perfluorinated linear C₁-C₄-alkyl and/or perfluorinated branched C₃-C₄-alkyl.

3. The electrolyte solution according to claim 1 or 2, wherein R² and R³ are a linear C₂-C₅-ether, preferably a linear C₂-C₄-ether selected from the group comprising -CH₂-O-CH₃, - CH₂-O-C₂H₅, -C₂H₄-O-CH₃, -C₂H₄-O-C₂H₅, -CH₂-O-C₃H₇, -C₃H₆-O-CH₃, preferably selected from the group comprising -CH₂-O-CH₃, -CH₂-O-C₂H₅ und/or -C₂H₄-O-CH₃.

4. The electrolyte solution according to any of the preceding claims, wherein R² and R³ are a branched C₄-C₇-ether, preferably selected from the group comprising -CH₂-O-CH(CH₃)₂, -CH(CH₃)-CH₂-O-CH₃, -C₂H₄-O-CH(CH₃)₂, -CH(CH₃)-CH₂-O-CH(CH₃)₂ and/or -CH(CH₃)-CH₂-O-C₂H₅.

5. The electrolyte solution according to any of the preceding claims, wherein the fluorinated sulfonamide is 1,1,2,2,3,3,4,4,4-nonafluoro-N,N-bis(2-methoxyethyl)butane-1-sulfonamide.

6. The electrolyte solution according to any of the preceding claims, wherein the electrolyte solution comprises the fluorinated sulfonamide in a range from ≥ 75 wt% to ≤ 100 UD 40670 / SAM:AL
wt%, preferably in a range of ≥ 85 wt% to ≤ 100 wt%, more preferably in a range of ≥ 90 wt% to ≤ 100 wt%, referring to a total amount of the electrolyte solvent of 100 wt%.

7. The electrolyte solution according to any of the preceding claims, wherein the electrolyte solution comprises at least one non-fluorinated organic carbonate solvent selected from the group comprising ethylene carbonate, ethyl methyl carbonate, propylene carbonate, dimethyl carbonate, diethyl carbonate, and mixtures thereof.

8. The electrolyte solution according to any of the preceding claims, wherein the electrolyte salt is selected from the group comprising alkali or alkaline earth metal sulfonimide, methide, sulfonate or oxalatoborate salts.

9. An electrochemical energy storage device, particularly a lithium battery, a primary or secondary lithium-ion battery or lithium polymer battery or a lithium-ion capacitor, comprising an electrolyte solution according to any of the preceding claims.

10. A method for preventing aluminum current collector corrosion in an electrochemical energy storage device, the method comprising the step of using an electrolyte solution comprising an electrolyte salt and an electrolyte solvent, wherein the electrolyte solvent comprises at least one fluorinated sulfonamide according to the general formula (1) R¹-SO₂-NR²R³ wherein:
R¹ is selected from the group comprising fluorinated linear C₁-C₄-alkyl, branched C₃-C₄-alkyl, linear C₂-C₄-ether and/or branched C₃-C₄-ether;
R², R³ are selected, the same or independently of the other, from the group comprising linear C₂-C₅-ether and/or branched C₃-C₁₀-ether.

11. The method according to claim 10, wherein R² and R³ are a linear C₂-C₅-ether, preferably a linear C₂-C₄-ether selected from the group comprising -CH₂-O-CH₃, -CH₂-O-C₂H₅, -C₂H₄-O-CH₃, -C₂H₄-O-C₂H₅, -CH₂-O-C₃H₇, -C₃H₆-O-CH₃, preferably selected from the group comprising -CH₂-O-CH₃, -CH₂-O-C₂H₅ und/or -C₂H₄-O-CH₃.

12. The method according to claims 10 or 11, wherein the fluorinated sulfonamide is 1,1,2,2,3,3,4,4,4-nonafluoro-N,N-bis(2-methoxyethyl)butane-1-sulfonamide.

13. The method according to any of the claims 10 to 12, wherein the electrolyte solution comprises the fluorinated sulfonamide in a range from ≥ 75 wt% to ≤ 100 wt%, preferably in a range of ≥ 85 wt% to ≤ 100 wt%, more preferably in a range of ≥ 90 wt% to ≤ 100 wt%, referring to a total amount of the electrolyte solvent of 100 wt%.

14. Use of a fluorinated sulfonamide according to the general formula (1) R¹-SO₂-NR²R³, wherein:
R¹ is selected from the group comprising fluorinated linear C₁-C₄-alkyl, branched C₃-C₄-alkyl, linear C₂-C₄-ether and/or branched C₃-C₄-ether;
R², R³ are selected, the same or independently of the other, from the group comprising linear C₂-C₅-ether and/or branched C₃-C₁₀-ether,
for the prevention of aluminum current collector corrosion in an electrochemical energy storage device.

## Patentansprüche

1. Elektrolytlösung, umfassend ein Elektrolytsalz und ein Elektrolytlösungsmittel, wobei das Elektrolytlösungsmittel wenigstens ein fluoriertes Sulfonamid der allgemeinen Formel (1) R¹-SO₂-NR²R³ umfasst, wobei:
R¹ ausgewählt ist aus der Gruppe umfassend fluoriertes lineares C₁-C₄-Alkyl, verzweigtes C₃-C₄-Alkyl, linearen C₂-C₄-Ether und/oder verzweigten C₃-C₄-Ether;
R², R³ gleich oder unabhängig voneinander ausgewählt sind aus der Gruppe umfassend linearen C₂-C₅-Ether und/oder verzweigten C₃-C₁₀-Ether.

2. Elektrolytlösung gemäß Anspruch 1, wobei R¹ ausgewählt ist aus der Gruppe umfassend perfluoriertes lineares C₁-C₄-Alkyl und/oder perfluoriertes verzweigtes C₃-C₄-Alkyl.

3. Elektrolytlösung gemäß Anspruch 1 oder 2, wobei R² und R³ ein linearer C₂-C₅-Ether sind, vorzugsweise ein linearer C₂-C₄-Ether ausgewählt aus der Gruppe umfassend -CH₂-O-CH₃, -CH₂-O-C₂H₅, -C₂H₄-O-CH₃, -C₂H₄-O-C₂H₅, -CH₂-O-C₃H₇, -C₃H₆-O-CH₃, vorzugsweise ausgewählt aus der Gruppe umfassend -CH₂-O-CH₃, -CH₂-O-C₂H₅ und/oder -C₂H₄-O-CH₃.

4. Elektrolytlösung gemäß einem der vorstehenden Ansprüche, wobei R² und R³ ein verzweigter C₄-C₇-Ether sind, vorzugsweise ausgewählt aus der Gruppe umfassend -CH₂-O-CH(CH₃)₂, -CH(CH₃)-CH₂-O-CH₃, -C₂H₄-O-CH(CH₃)₂, -CH(CH₃)-CH₂-O-CH(CH₃)₂ und/oder -CH(CH₃)-CH₂-O-C₂H₅.

5. Elektrolytlösung gemäß einem der vorstehenden Ansprüche, wobei das fluorierte Sulfonamid 1,1,2,2,3,3,4,4,4-Nonafluor-N,N-bis(2-methoxyethyl)butan-1-sulfonamid ist.

6. Elektrolytlösung gemäß einem der vorstehenden Ansprüche, wobei die Elektrolytlösung das fluorierte Sulfonamid in einem Bereich von ≥ 75 Gew.-% bis ≤ 100 Gew.-% umfasst, vorzugsweise in einem Bereich von ≥ 85 Gew.-% bis ≤ 100 Gew.-%, bevorzugter in einem Bereich von ≥ 90 Gew.-% bis ≤ 100 Gew.-%, bezogen auf eine Gesamtmenge des Elektrolytlösungsmittels von 100 Gew.-%.

7. Elektrolytlösung gemäß einem der vorstehenden Ansprüche, wobei die Elektrolytlösung wenigstens ein nichtfluoriertes organisches Carbonatlösungsmittel ausgewählt aus der Gruppe umfassend Ethylencarbonat, Ethylmethylcarbonat, Propylencarbonat, Dimethylcarbonat, Diethylcarbonat und Gemische davon umfasst.

8. Elektrolytlösung gemäß einem der vorstehenden Ansprüche, wobei das Elektrolytsalz ausgewählt ist aus der Gruppe umfassend Alkali- oder Erdalkalimetallsulfonimid-, -methid-, -sulfonat- oder -oxalatoboratsalze.

9. Elektrochemische Energiespeichervorrichtung, vorzugsweise eine Lithiumbatterie, eine primäre oder sekundäre Lithiumionenbatterie oder Lithiumpolymerbatterie oder ein Lithiumionenkondensator, umfassend eine Elektrolytlösung gemäß einem der vorstehenden Ansprüche.

10. Verfahren zum Verhindern von Aluminiumstromabnehmer-Korrosion in einer elektrochemischen Energiespeichervorrichtung, wobei das Verfahren den Schritt der Verwendung einer Elektrolytlösung umfassend ein Elektrolytsalz und ein Elektrolytlösungsmittel umfasst, wobei das Elektrolytlösungsmittel wenigstens ein fluoriertes Sulfonamid der allgemeinen Formel (1) R¹-SO₂-NR²R³ umfasst, wobei:
R¹ ausgewählt ist aus der Gruppe umfassend fluoriertes lineares C₁-C₄-Alkyl, verzweigtes C₃-C₄-Alkyl, linearen C₂-C₄-Ether und/oder verzweigten C₃-C₄-Ether;
R², R³ gleich oder unabhängig voneinander ausgewählt sind aus der Gruppe umfassend linearen C₂-C₅-Ether und/oder verzweigten C₃-C₁₀-Ether.

11. Verfahren gemäß Anspruch 10, wobei R² und R³ ein linearer C₂-C₅-Ether sind, vorzugsweise ein linearer C₂-C₄-Ether ausgewählt aus der Gruppe umfassend -CH₂-O-CH₃, -CH₂-O-C₂H₅, -C₂H₄-O-CH₃, -C₂H₄-O-C₂H₅, -CH₂-O-C₃H₇, -C₃H₆-O-CH₃, vorzugsweise ausgewählt aus der Gruppe umfassend -CH₂-O-CH₃, -CH₂-O-C₂H₅ und/oder -C₂H₄-O-CH₃.

12. Verfahren gemäß der Ansprüche 10 oder 11, wobei das fluorierte Sulfonamid 1,1,2,2,3,3,4,4,4-Nonafluor-N,N-bis(2-methoxyethyl)butan-1-sulfonamid ist.

13. Verfahren gemäß einem der Ansprüche 10 bis 12, wobei die Elektrolytlösung das fluorierte Sulfonamid in einem Bereich von ≥ 75 Gew.-% bis ≤ 100 Gew.-% umfasst, vorzugsweise in einem Bereich von ≥ 85 Gew.-% bis ≤ 100 Gew.-%, bevorzugter in einem Bereich von ≥ 90 Gew.-% bis ≤ 100 Gew.-%, bezogen auf eine Gesamtmenge des Elektrolytlösungsmittels von 100 Gew.-%.

14. Verwendung eines fluorierten Sulfonamids der allgemeinen Formel (1) R¹-SO₂-NR²R³, wobei:
R¹ ausgewählt ist aus der Gruppe umfassend fluoriertes lineares C₁-C₄-Alkyl, verzweigtes C₃-C₄-Alkyl, linearen C₂-C₄-Ether und/oder verzweigten C₃-C₄-Ether;
R², R³ gleich oder unabhängig voneinander ausgewählt sind aus der Gruppe umfassend linearen C₂-C₅-Ether und/oder verzweigten C₃-C₁₀-Ether,
zum Verhindern von Aluminiumstromabnehmer-Korrosion in einer elektrochemischen Energiespeichervorrichtung.

## Revendications

1. Solution d'électrolyte comprenant un sel d'électrolyte et un solvant d'électrolyte, dans laquelle le solvant d'électrolyte comprend au moins un sulfonamide fluoré selon la formule générale (1) R¹-SO₂-NR²R³ dans laquelle :
R¹ est choisi dans le groupe comprenant alkyle en C₁-C₄ linéaire, alkyle en C₃-C₄ ramifié, éther en C₂-C₄ linéaire et/ou éther en C₃-C₄ ramifié fluorés ;
R², R³ sont choisis, identiques ou indépendamment l'un de l'autre, dans le groupe comprenant éther en C₂-C₅ linéaire et/ou éther en C₃-C₁₀ ramifié.

2. Solution d'électrolyte selon la revendication 1, dans laquelle R¹ est choisi dans le groupe comprenant alkyle en C₁-C₄ linéaire perfluoré et/ou alkyle en C₃-C₄ ramifié perfluoré.

3. Solution d'électrolyte selon la revendication 1 ou 2, dans laquelle R² et R³ sont un éther en C₂-C₅ linéaire, de préférence un éther en C₂-C₄ linéaire choisi dans le groupe comprenant -CH₂-O-CH₃, -CH₂-O-C₂H₅, -C₂H₄-O-CH₃, -C₂H₄-O-C₂H₅, -CH₂-O-C₃H₇, -C₃H₆-O-CH₃, de préférence choisi dans le groupe comprenant -CH₂-O-CH₃, -CH₂-O-C₂H₅ et/ou -C₂H₄-O-CH₃.

4. Solution d'électrolyte selon l'une quelconque des revendications précédentes, dans laquelle R² et R³ sont un éther en C₄-C₇ ramifié, de préférence choisi dans le groupe comprenant -CH₂-O-CH(CH₃)₂, -CH(CH₃)-CH₂-O-CH₃, -C₂H₄-O-CH(CH₃)₂, -CH(CH₃)-CH₂-O-CH(CH₃)₂ et/ou -CH(CH₃)-CH₂-O-C₂H₅.

5. Solution d'électrolyte selon l'une quelconque des revendications précédentes, dans laquelle le sulfonamide fluoré est le 1,1,2,2,3,3,4,4,4-nonafluoro-N,N-bis(2-méthoxyéthyl)butane-1-sulfonamide.

6. Solution d'électrolyte selon l'une quelconque des revendications précédentes, la solution d'électrolyte comprenant le sulfonamide fluoré dans une plage de ≥ 75 % en poids à ≤ 100 % en poids, de préférence dans une plage de ≥ 85 % en poids à ≤ 100 % en poids, plus préférablement dans une plage de ≥ 90 % en poids à ≤ 100 % en poids, par rapport à une quantité totale du solvant d'électrolyte de 100 % en poids.

7. Solution d'électrolyte selon l'une quelconque des revendications précédentes, la solution d'électrolyte comprenant au moins un solvant carbonate organique non fluoré choisi dans le groupe comprenant le carbonate d'éthylène, le carbonate d'éthylméthyle, le carbonate de propylène, le carbonate de diméthyle, le carbonate de diéthyle, et des mélanges de ceux-ci.

8. Solution d'électrolyte selon l'une quelconque des revendications précédentes, dans laquelle le sel d'électrolyte est choisi dans le groupe comprenant un sel de sulfonimide, méthide, sulfonate ou oxalatoborate de métal alcalin ou alcalino-terreux.

9. Dispositif de stockage d'énergie électrochimique, en particulier une batterie au lithium, une batterie au lithium-ion primaire ou rechargeable ou une batterie au lithium-polymère ou un condensateur au lithium-ion, comprenant une solution d'électrolyte selon l'une quelconque des revendications précédentes.

10. Procédé de prévention de la corrosion d'un collecteur de courant en aluminium dans un dispositif de stockage d'énergie électrochimique, le procédé comprenant l'étape d'utilisation d'une solution d'électrolyte comprenant un sel d'électrolyte et un solvant d'électrolyte, le solvant d'électrolyte comprenant au moins un sulfonamide fluoré selon la formule générale (1) R¹-SO₂-NR²R³ dans laquelle :
R¹ est choisi dans le groupe comprenant alkyle en C₁-C₄ linéaire, alkyle en C₃-C₄ ramifié, éther en C₂-C₄ linéaire et/ou éther en C₃-C₄ ramifié fluorés ;
R², R³ sont choisis, identiques ou indépendamment l'un de l'autre, dans le groupe comprenant éther en C₂-C₅ linéaire et/ou éther en C₃-C₁₀ ramifié.

11. Procédé selon la revendication 10, dans lequel R² et R³ sont un éther en C₂-C₃ linéaire, de préférence un éther en C₂-C₄ linéaire choisi dans le groupe comprenant -CH₂-O-CH₃, -CH₂-O-C₂H₅, -C₂H₄-O-CH₃, -C₂H₄-OC₂H₅, -CH₂-O-C₃H₇, -C₃H₆-O-CH₃, de préférence choisi dans le groupe comprenant -CH₂-O-CH₃, -CH₂-O-C₂H₅ et/ou -C₂H₄-O-CH₃.

12. Procédé selon les revendications 10 ou 11, dans lequel le sulfonamide fluoré est le 1,1,2,2,3,3,4,4,4-nonafluoro-N,N-bis(2-méthoxyéthyl)butane-1-sulfonamide.

13. Procédé selon l'une quelconque des revendications 10 à 12, dans lequel la solution d'électrolyte comprend le sulfonamide fluoré dans une plage de ≥ 75 % en poids à ≤ 100 % en poids, de préférence dans une plage de ≥ 85 % en poids à ≤ 100 % en poids, plus préférablement dans une plage de ≥ 90 % en poids à ≤ 100 % en poids, par rapport à une quantité totale du solvant d'électrolyte de 100 % en poids.

14. Utilisation d'un sulfonamide fluoré selon la formule générale (1) R¹-SO₂-NR²R³ dans laquelle :
R¹ est choisi dans le groupe comprenant alkyle en C₁-C₄ linéaire, alkyle en C₃-C₄ ramifié, éther en C₂-C₄ linéaire et/ou éther en C₃-C₄ ramifié fluorés;
R², R³ sont choisis, identiques ou indépendamment l'un de l'autre, dans le groupe comprenant éther en C₂-C₅ linéaire et/ou éther en C₃-C₁₀ ramifié,
pour la prévention de la corrosion d'un collecteur de courant en aluminium dans un dispositif de stockage d'énergie électrochimique.
